# EUROPEAN PATENT SPECIFICATION

(11) **EP 2 063 794 B1**
(45) Date of publication and mention of the grant of the patent: **29.09.2010**
(21) Application number: 07813568.8
(22) Date of filing: 31.07.2007
(51) Int. Cl.: A61B 17/70

(54) **MULTIPLE ROD SYSTEM WITH ELASTIC ROD-CONTAINING MEANS**
MULTISTANGENSYSTEM MIT ELASTISCHEN STANGEN ENTHALTENDEM MITTEL
SYSTÈME À TIGES MULTIPLES AYANT DES MOYENS ÉLASTIQUES CONTENANT DES TIGES

(30) Priority: 01.08.2006 FR 0607038
(43) Date of publication of application: 03.06.2009
(73) Proprietor: Warsaw Orthopedic, Inc., Warsaw, Indiana 46581 (US)
(72) Inventor: VARGA, Peter, 1126 Nagy Jeno Utca 8 (HU); NAYET, Jerome, 01630 Saint Genis Pouilly (FR); GOURNAY, Jose, 01170 Gex (FR)
(74) Representative: Hutchinson, Glenn Stanley
(86) International application number: PCT/US2007/074789
(87) International publication number: WO 2008/016892

(56) References cited:
- WO-A-2004/091413
- FR-A- 2 780 269
- FR-A- 2 859 376
- US-A1- 2005 080 419
- US-A1- 2005 277 927

## Description

In order to correct deformations of the spine such as scoliosis, spondylolisthesis, lordosis or kyphosis, it is known to use devices comprising one or more rods made generally of metal, for example steel, which extend over at least part of the length of the spine and are made integral with one or two rows of anchoring elements (or implants) implanted on the spine. Such devices are described in the document US-A-5,005,562, for example.

These anchoring elements are often formed by screws which are implanted in a vertebra, or by hooks which are assigned an anchoring point on the lamina, pedicle or transverse process of a vertebra.

The head of these anchoring elements is designed to support at least one U-shaped channel which is perpendicular to the axis of the element and in which a rod is inserted. The U-shaped channel is delimited by two lateral branches. On their surface facing into the channel, these lateral branches in most cases have threads constituting an internal threading arrangement that permits insertion and blocking of a plug of generally cylindrical shape. This plug, when it is tightened, has the function of pressing the rod against the bottom of the channel and of keeping the rod there after the device has been fitted.

When the corrective device is being put in place, the rod is inserted into the U-shaped channels of the heads of the anchoring elements which have been fixed beforehand on the vertebrae, and the plugs are then screwed into the channels by means of a suitable screwdriver.

In cases where it is necessary to correct a severe deformation of the spine, quite considerable mechanical stresses, for example in flexion and/or in rotation, have to be imposed on the rod when introducing it into the channels of the heads of the anchoring elements. In the zones where the deformation is at its maximum, the rod is at a distance from the implant, and it is necessary to use a specific heavy and bulky instrument to bring it between the branches of the U-shaped channel. This maneuver increases the operating time and sometimes necessitates an extension of the incisions made on the patient. The relatively high rigidity of the rod makes it difficult to deform in order to insert it into the heads of the anchoring elements. Moreover, the stresses generated upon introduction of the rod are directly transmitted to the bone-anchoring elements, thus compromising their long-term stability.

It has been proposed in documents GB-A-2 294 394, WO-A-02/41 797 and WO-A-02/085 217 to replace the single rod of large diameter in these devices with a plurality of rods of small diameter which the surgeon inserts one after another into channels defined by the heads of the anchoring elements. A threaded plug holds the rods in their seat. These rods are inserted either longitudinally (GB-A-2 294 394 and WO-A-02/085 217) or laterally (WO-A-02/41 797) into the heads of the anchoring elements. On account of the small diameter of each rod, it is easier for the surgeon to shape them so as to be able to insert them into the heads of the anchoring elements, and experience has shown that the cumulative effects of these multiple rods of small diameter, pressed against one another inside the heads by the plugs, are comparable to those of the customary single rods of large diameter.

In the document WO-A-2005/058173, the Applicant has also proposed designing the heads of the anchoring elements in such a way that the rods are stacked in one or more columns, so as to give the surgeon the possibility of better adapting the properties of the corrective device depending on the specific needs of the patient.

A difficulty encountered by the surgeon when fitting the device in place is that when the heads of the anchoring elements are designed to permit longitudinal insertion of the rods, there is a risk that rods already inserted will escape from their seats before the threaded plug is put in place, in particular when they are subjected to considerable torsion. From this point of view, the lateral insertion of the rods proposed by WO-A-02/417797, with the plug pre-fitted on the head, provides an improvement, but it complicates the step of insertion of the rods because of the reduced width of the insertion slit and because of the lateral positioning thereof on the head. In particular, at the insertion site, it is necessary for the rod to have a substantially rectilinear configuration and, in particular, to be parallel to the slit. Insertion is therefore very difficult in areas where the rod has a substantial curvature.

FR2859376, FR2780269, US2005/277927 and US2005/080419 disclose implants capable of retaining a single large diameter rod in the head thereof. WO2004/091413 discloses a bone fixation element capable of retaining a plurality of small diameter rods using blocking elements.

The object of the invention is to propose a design of the heads of the anchoring elements which permits easy insertion of rods of small diameter and effectively limits the possibility of the rods escaping from their seat after they have been inserted.

To this end, the subject of the invention is a bone-anchoring element, such as a screw, for a device for correcting deformations of a patient's spine, which is intended to be implanted on a vertebra and whose head comprises a receptacle for a plurality of rods of small diameter, with a channel which opens out on the upper edge of the head and is defined by two lateral branches whose inner faces are provided with a thread for the insertion and blocking of a threaded plug, characterized in that it includes at least one elastic element which locally limits the width of the channel and prevents or impedes the expulsion of the rods from the receptacle when they are inserted in the head and are not yet blocked by the plug.

Features of the invention are defined in the appended claims.

The bone-anchoring element can include at least one seat formed in the head facing the channel and the receptacle, and a U-shaped elastic component which can be inserted into said seat and comprises two vertical branches connected via a substantially semi-circular lower part whose internal dimensions and shape correspond substantially to those of the receptacle, said vertical branches each comprising a protuberance that locally reduces the width of the space separating the branches.

The upper lateral edges of said seat can be curved in toward the inside of the seat in such a way as to reduce its width at its entrance, and the upper parts of the branches of the U-shaped component comprise notches.

Said elastic element can be formed by portions of the branches of the head.

The invention also has as its subject a device for correcting deformations of the spine, comprising bone-anchoring elements, such as screws, which are implanted on the vertebrae of the patient, and a rod which is inserted in the heads of said screws, characterized in that at least one of said elements is of the above type.

Said rod can be made up of a plurality of rods of small diameter.

As will have been understood, the invention lies in providing the head of a bone-anchoring element for longitudinal insertion of the rods with at least one elastic element that locally limits the width of the insertion slit.

This elastic element can be formed in particular by a separate element with a general U-shape which narrows at its open end and which can be inserted in the head of the anchoring element at one of its longitudinal openings for insertion of the rods.

It can also be formed by the anchoring element's actual head whose configuration with at least one longitudinal opening for insertion of the rods presents said narrowed U-shape at its open end, and whose mechanical properties permit elastic spreading of the edges of the opening during insertion of a rod whose diameter slightly exceeds the nominal width of the opening.

The bone-anchoring element according to the invention can be a mono-axial screw, as in the example described and shown below. However, it can also be a multi-axial screw, or a hook, or any type of connector. It can be implanted using an anterior approach or a posterior approach.

The invention will be better understood on reading the following description in which reference is made to the attached figures, of which:
- Figure 1 shows a front view of an example of a bone-anchoring screw according to the invention, equipped with its U-shaped elastic component for holding the rods in place after their insertion;

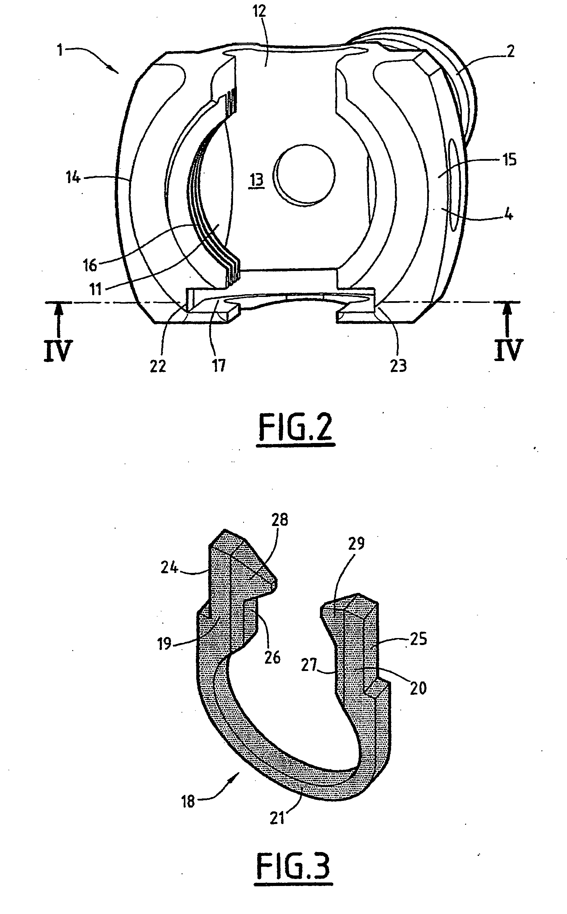

- Figure 2 shows a plan view of said screw, without its U-shaped component;
- Figure 3 shows a perspective view of said U-shaped component;
- Figures 4, 5 and 6 show perspective views, in cross section along IV-IV, of the screw with the U-shaped component in three positions of insertion;
- Figure 7 is a schematic representation of the procedure for inserting the rods into the screw;
- Figure 8 is a schematic representation of the state of the screw and of the U-shaped component after the insertion of the rods;
- Figure 9 is a perspective view of the screw and of the rods after the plug has been tightened and is holding the rods in place.

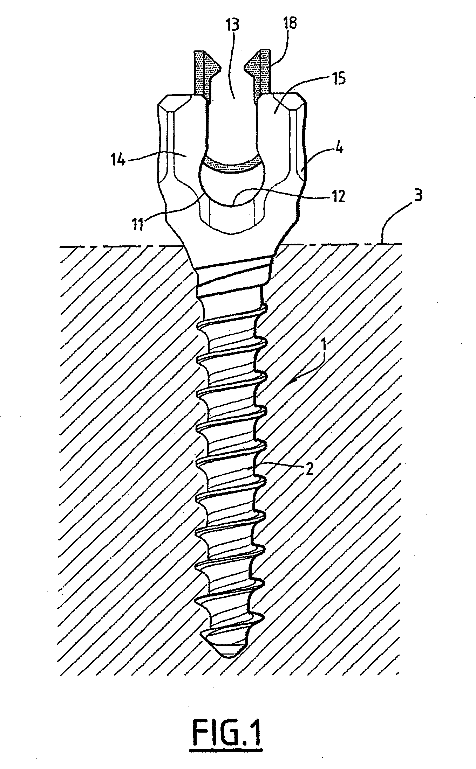

Figure 1 shows a monoaxial bone-anchoring screw 1 of a device for correcting deformations of the spine in accordance with one illustrative embodiment of the invention. This screw 1 comprises, as is customary, an externally threaded lower part 2 permitting fixation of the screw 1 in a vertebra whose contour 3 is indicated by a broken line.

The screw 1 also comprises a head 4 into which independent rods 5, 6, 7, 8, 9, 10 of relatively small diameter, for example of the order of 1.3 to 3 mm, are inserted. These rods 5-10, which are typically (but not necessarily) of metal, constitute the longitudinal element of the corrective device extending along that part of the spine whose curvature is to be corrected, and for this purpose they fit into the heads of other screws which are similar to the screw 1 and which are implanted in the vertebrae of the area of the spine to be treated. The rods 5-10 are received in a receptacle 11 which is formed in the head 4 and whose bottom 12 has a substantially cylindrical surface. The receptacle 11 opens out toward the top by way of a channel 13 of U-shaped cross section defined by lateral branches 14, 15 of the head 4, the inner faces of these branches 14, 15 being provided with a thread 16 for the insertion and blocking of a threaded plug 30. All these features are customary for bone-anchoring screws that are intended to receive a single rod of large diameter or multiple rods of small diameter 5-10 of an appliance for correcting deformations of the spine.

According to one embodiment of the invention, the head 4 comprises, facing the receptacle 11 and the channel 13, a seat 17 in which an elastic U-shaped component 18 can be inserted. The latter is made up of two vertical branches 19, 20 which are connected via a substantially semicircular lower portion 21.

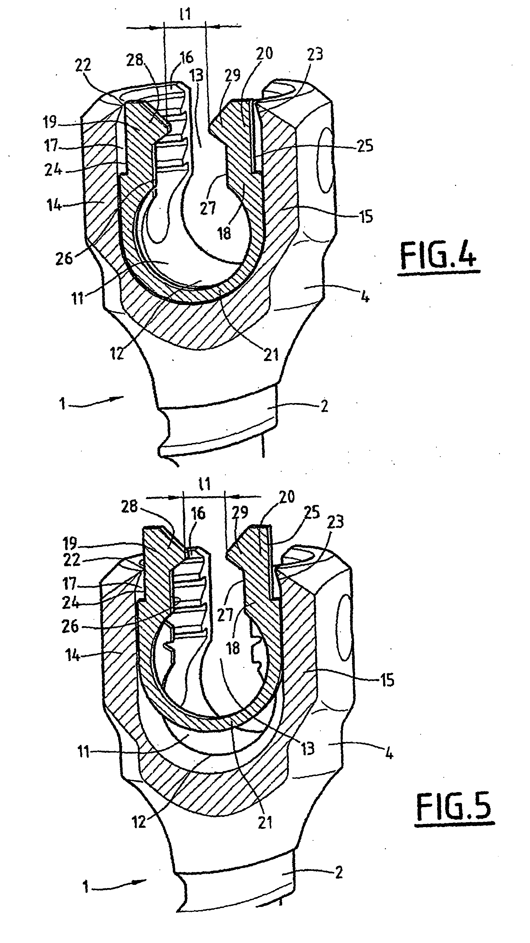

Figure 4 shows the head 4 in more detail, in cross section along IV-IV, with the U-shaped elastic component 18 inserted fully in the seat 17. It will be seen that the lower part 21 of the elastic component 18 has a shape and internal dimensions that correspond substantially to those of the receptacle 11, so as to leave the entrance to the receptacle 11 free. It will also be seen that the upper lateral edges 22, 23 of the seat 17 are curved slightly in toward the inside of the seat 17 in such a way as to reduce the latter's width at its entrance. This feature cooperates with notches 24, 25 formed on the upper part of the outer face of the branches 19, 20 of the U-shaped component 18, as will be explained below.

On the upper parts of the inner faces 26, 27 of its branches 19, 20, the U-shaped component 18 has beveled protuberances 28, 29 which in this area reduce the width of the space separating the branches 19, 20 to a first value 1₁. In accordance with the invention, this feature makes it easier to fit the rods 5-10 of the device for correcting deformations of the spine, as will be described below.

Figure 5 shows the head 4 in cross section along IV-IV, with the elastic U-shaped component 18 not fully inserted in the seat 17, but such that the upper lateral edges 22, 23 of the seat 17 are situated opposite the external notches 24, 25 of the branches 19, 20 of the U-shaped component 18. In this configuration, the space separating the branches 19, 20 has the same minimum width 1₁, at the area of the protuberances 28, 29, as when the U-shaped component 18 is fully inserted in the seat 17.

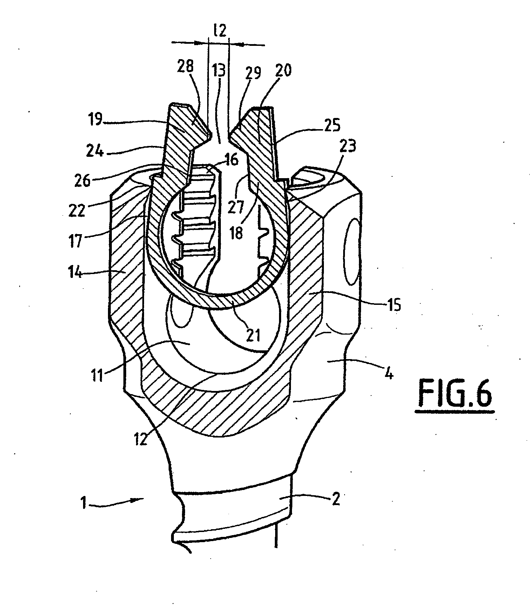

Figure 6 shows the head 4 in cross section along IV-IV, with the elastic U-shaped component 18 not fully inserted in the seat 17, so that the upper lateral edges 22, 23 of the seat 17 are situated below the notches 24, 25 of the branches 19, 20 of the U-shaped component 18. They thus exert a pressure against the branches 19, 20 which tends to reduce to a second value 1₂, less than 1₁, the minimum width of the space separating the branches 19, 20 in the area of the protuberances 28, 29.

The device for correcting deformations of the spine according to the invention is fitted in place in the following way.

The surgeon implants on the patient's vertebrae 3 the various screws 1 that are used for fixing the device, and he then inserts the rods 5-10 of diameter "d" one by one into the heads 4 of the screws 1.

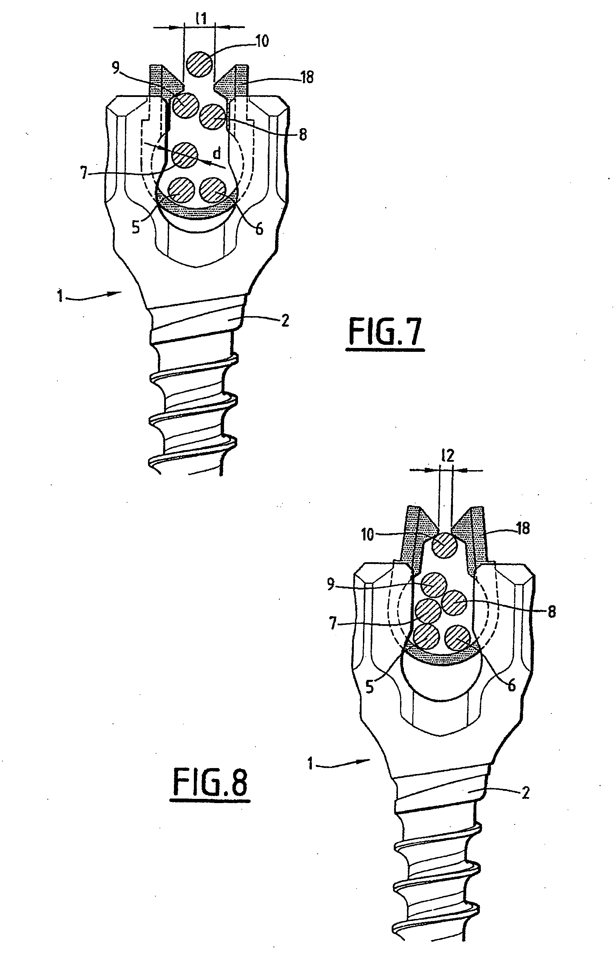

For this purpose, as is shown in Figure 7, he can place the U-shaped components 18 in the position shown in Figure 5, so that the spaces separating the branches 19, 20 of the U-shaped components 18 are given the first minimum width 1₁ mentioned above. This l₁ must be greater than the diameter "d" of the rods 5-10 in order to permit insertion of the rods 5-10, but only just, so as to make it difficult for the rods 5-10 to escape after their insertion, in cases where the deformations of the rods 5-10 would have a tendency to provoke this escape. The bevels of the protuberances 28, 29 make it easy for the rods 5-10 to enter the interior space of the U-shaped component 18.

As is shown in Figure 8, escape of the rods 5-10 can be totally prevented if, after inserting all of them (or only some of them), the U-shaped components 18 are placed in the position shown in Figure 6, so that the spaces separating their branches 19, 20 are given the second minimum width 1₂ mentioned above, 1₂ being smaller than the diameter "d" of the rods 5-10. In many cases in fact, the rods 5-10 will have a tendency to leave the receptacle 11 under the effect of their own energy and due to their elastic deformation. In doing so, they themselves tend to drive the U-shaped component 18 upward, and thus bring its branches 19, 20 closer together. There is therefore an effect whereby the rods 5-10 are automatically captured by the U-shaped component 18.

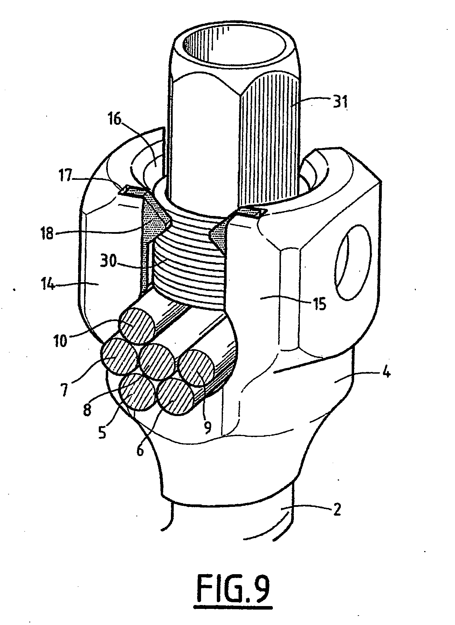

Once all the rods 5-10 are placed in the head 4, where they are held by the U-shaped component 18, the surgeon can place a threaded plug 30 between the branches 14, 15 of the head 4 and tighten it so as to press the rods 5-10 against the bottom 12 of the receptacle 11. The pressure exerted on the rods 5-10 by the plug 30 during this tightening also causes the U-shaped component 18 to descend inside the seat 17, so that, at the end of the tightening procedure, it no longer emerges above the upper edge of the head 4. This therefore gives the situation illustrated in Figure 9. In the example shown, the plug 30 comprises, as is known, a divisible upper part 31 which will separate from the lower part at the end of the tightening procedure, when the force applied exceeds a predetermined value. The plug 30 tightened in this way will therefore no longer protrude above the head 4.

It is generally preferable not to fit and tighten the plugs 30 of the screws 1 of the device until after the rods 5-10 have been inserted in the heads 4 in all the screws 1 of the device. The rods 5-10 are easier to fit in place in this way, because the rods 5-10 then advantageously retain possibilities of movement and of bending, which possibilities would be more limited after the plug 30 has been tightened. The presence of the U-shaped components 18 ensures that, during this fitting, rods 5-10 that have already been inserted in one head 4 do not escape from the latter when the surgeon is maneuvering them in order to insert them in another head 4.

Alternatively, two seats 17 could be formed in the head 4, that is to say one facing each entrance of the receptacle 11, so as to be able to insert two elastic U-shaped components 18 in the head 4 and thus improve still further the holding of the rods 5-10 during installation of the corrective device.

The invention has been described in terms of its preferred application, which is the fitting of corrective devices whose rod is composed of multiple rods 5-10 of small diameter. It goes without saying, however, that it can also be used for devices comprising a single rod of greater diameter, if a U-shaped component 18 of suitable dimensions is used for this purpose.

As the heads 4 of the screws 1 according to the invention are practically identical in form and dimensions to those of the customary screws (except for a possible slight added thickness of the head 4 in the region of the seat 17), they can be used on any corrective device in place of the customary screws. If the surgeon does not wish to use the possibility of maintaining the rod or rods 5-10 in the heads 4 of the screws 1, he simply does not need to insert U-shaped components 18 in the seats 17. The product range of the bone-anchoring elements can thus be simple, the elements with a seat 17 according to the invention being able to be used without any problem as a substitute for the conventional elements that do not have this seat.

An advantage of this situation is that the screws 1 according to the invention can be reused if a modification of the corrective device, of which they form part, proves expedient at a subsequent stage of the patient's treatment. For example, if the surgeon wishes to replace the initial single rod with a multi-part rod 5-10 better adapted to correcting the deformation of the spine in a precise area, as could prove necessary after a first rough correction procured by the initial rod, it suffices to substitute the rods and to add U-shaped components 18 to the heads 4 of the screws 1. The reverse change from a multi-part rod 5-10 to a single rod would also be possible by removing the U-shaped components 18.

Compared to the configuration of the screws in document WO-A-02/41797, in which the channel for entry of the rods into the receptacle is formed on the lateral part of the heads, the invention particularly affords the advantage of not requiring that the rod 5-10 being inserted has a relatively rectilinear portion along a length corresponding more or less to the diameter of the head 4. Insertion of the rods 5-10 is therefore simpler and requires fewer instruments. Moreover, the screw 1 according to the invention is more versatile in its application, since its entry channel 13 accepts rods of very different diameters. In the screws in WO-A-02/41797, the width of the slit limits the diameter of the rods that can be inserted while benefiting from the function of their being held in place inside the head before being blocked. In the screws 1 of the invention, the same screw 1 can receive and hold rods 5-10 of different diameters; it suffices to change the U-shaped component 18, choosing a width 1₁ of the space separating its branches 19, 20 that is suitable for the diameter of the rods 5-10 being used.

A variant of the invention would consist in omitting the seat 17 and the elastic U-shaped component 18 and in giving the branches 14, 15 of the head 4, in their portions defining one of the entries (or both entries) of the channel 13, a configuration and an elasticity that are comparable to those of the U-shaped component 18, as have been described. In doing this, the advantages associated with the interchangeability of the U-shaped component 18 are lost; nevertheless, the main function of holding the rods 5-10 in place is ensured while at the same time reducing the number of component parts of the device.

As a non-limiting example, it is possible to imagine a screw 1 and a U-shaped component having the following main characteristics. For a head 4 with a width of 13.4 mm, it is possible to provide a U-shaped component having a maximum width of 8 mm and a thickness of 0.9 mm. The width l₁ can vary between 1.5 and 6.35 mm, depending on the diameter of the rods 5-10 used. The bottom 12 of the receptacle 11 has a radius of 3.4 mm, which is also that of the inner edge of the semi-circular portion 21 of the U-shaped component 18. The latter can be made of any biocompatible elastic material: titanium, austenitic stainless steel, shape-memory alloy Cr-Co, a polymer such as PEEK, etc.

The invention can also be applied to the case of document WO-A-2005/058173 where the head of the bone-anchoring element comprises several compartments for organizing the rods in separate columns, this requiring adaptations obvious to a person skilled in the art.

It is not absolutely necessary for all the bone-anchoring elements of an apparatus for correcting deformations of the spine according to the invention to be of the type according to the invention. It may sometimes suffice to provide such elements only in one or more zones where the rod or rods of the apparatus are most strongly deformed.

## Claims

1. A bone-anchoring element, such as a screw (1), for a device for correcting deformations of a patient's spine, which is intended to be implanted on a vertebra (3) and whose head (4) comprises a receptacle (11) for one or more rods (5-10) of small diameter, with a channel (13) which opens out on the upper edge of the head (4) and is defined by two lateral branches (14, 15) whose inner faces are provided with a thread (16) for the insertion and blocking of a threaded plug (30), **characterised in that** the bone-anchoring element includes at least one elastic element which locally limits the width of the channel (13) and prevents or impedes the expulsion of the rods (5-10) from the receptacle (11) when they are inserted in the head (4) and are not yet blocked by the plug (30).

2. The bone-anchoring element as claimed in claim 1, including at least one seat (17) formed in the head (4) facing the channel (13) and the receptacle (11), and a U-shaped elastic component (18) which can be inserted into said seat (17) and comprises two vertical branches (19, 20) connected via a substantially semi-circular lower part (2) whose internal dimensions and shape correspond substantially to those of the receptacle (11), said vertical branches (19, 20) each comprising a protuberance (28, 29) that locally reduces the width of the space separating the branches (19, 20).

3. The bone-anchoring element as claimed in claim 2, wherein the upper lateral edges (22, 23) of said seat (17) are curved in toward the inside of the seat (17) in such a way as to reduce its width at its entrance, and wherein the upper parts of the branches (19, 20) of the U-shaped component (18) comprise notches (24, 25).

4. The bone-anchoring element as claimed in claim 1, wherein said elastic element is formed by portions of the branches (14, 15) of the head (4).

5. The bone-anchoring element as claimed in claim 1, wherein said local limitation of the width of the channel (13) has a length corresponding to less than the diameter of the head (4)

6. A device for correcting deformations of the spine, comprising bone-anchoring elements which are implanted on the vertebrae (3) of the patient, and a plurality of rods (5-10) of small diameter which are inserted in the heads (4) of said elements, wherein at least one of said elements is of the type as claimed in one of claims 1 to 4.

7. The device as claimed in claim 6, further comprising a threaded plug (30) for blocking said rods in the head (4) of said at least one of said elements of the type claimed in one of claims 1 to 4.

## Patentansprüche

1. Knochenverankerungselement, wie zum Beispiel eine Schraube (1), für eine Vorrichtung zur Korrektur von Deformationen der Wirbelsäule eines Patienten,wobei das Element für eine Implantation an einem Wirbel (3) vorgesehen ist, und wobei der Kopf (4) des Elements eine Aufnahmeeinrichtung (11) für eine oder mehrere Stangen (5-10) mit kleinem Durchmesser umfasst, mit einem Kanal (13), der sich an dem oberenRand des Kopfes (4) nach außen öffnet und durch zwei laterale Verzweigungen (14, 15) definiert ist, deren Innenseiten mit einem Gewinde (16) zum Einführen und Verriegeln eines Gewindestopfens (30) versehen sind, **dadurch gekennzeichnet, dass** das Knochenverankerungselementmindestens ein elastisches Element aufweist, das die Breite des Kanals (13) lokal begrenzt und das Ausstoßen der Stangen (5-10) aus der Aufnahmeeinrichtung (11) verhindert oder behindert, wenn diese in den Kopf (4) eingeführt werden und noch nicht durch den Stopfen (30) verriegelt werden.

2. Knochenverankerungselement nach Anspruch 1, mit mindestens einem Sitz (17), der in dem Kopf (4) ausgebildet ist und zu dem Kanal (13) und der Aufnahmeeinrichtung (11) ausgerichtet ist, und mit einer U-förmigen elastomeren Komponente (18), die in den Sitz (17) eingeführt werden kann und zwei vertikale Verzweigungen (19, 20) umfasst, die über ein im Wesentlichen halbkreisförmiges unteres Teil (2) verbundensind, dessen interne Abmessungen und Form im Wesentlichen den Abmessungen und der Form der Aufnahmeeinrichtung (11) entsprechen, wobei die genannten vertikalen Verzweigungen (19, 20) jeweils eine Protuberanz (28, 29) umfassen, die lokal die Breite des Zwischenraums reduziert, der die Verzweigungen (19, 20) voneinander trennt.

3. Knochenverankerungselementnach Anspruch 2, wobei die oberenlateralen Kanten (22, 23) des genannten Sitzes (17) in Richtung der Innenseite des Sitzes (17) derart gekrümmt sind, dass sich dessen Breite an dessen Eingang reduziert, und wobei die oberenTeile der Verzweigungen (19, 20) der U-förmigen Komponente (18) Kerben (24, 25) umfassen.

4. Knochenverankerungselementnach Anspruch 1, wobei das genannte elastische Element durch Teilstückeder Verzweigungen (14, 15) des Kopfes (4) gebildet wird.

5. Knochenverankerungselementnach Anspruch 1, wobei die genannte lokale Begrenzung der Breite des Kanals (13) eine Länge aufweist, die kleiner ist als der Durchmesser des Kopfes (4).

6. Vorrichtung zur Korrektur von Deformationen der Wirbelsäule, wobei die Vorrichtung Knochenverankerungselemente umfasst, die an den Wirbeln (3) des Patientenimplantiert werden, sowie eine Mehrzahl von Stangen (5-10) mit kleinem Durchmesser, die in die Köpfe (4) der genannten Elemente eingeführt werden, wobei mindestens eines der genannten Elemente der in einem der Ansprüche 1 bis 4 beanspruchten Art entspricht.

7. Vorrichtung nach Anspruch 6, wobei diese ferner einen Gewindestopfen (30) zur Verriegelung der genannten Stangen (4) des genannten mindestens einen der genannten Elemente der in einem der Ansprüche 1 bis 4 beanspruchten Art umfasst.

## Revendications

1. Élément d'ancrage d'os, tel qu'une vis (1), pour un dispositif pour corriger les déformations de la colonne vertébrale d'un patient, qui est destiné à être implanté sur une vertèbre (3) et dont la tête (4) comprend un réceptacle (11) pour une ou plusieurs tiges (5-10) de petit diamètre, avec un canal (13) qui s'ouvre sur le bord supérieur de la tête (4) et est défini par deux branches latérales (14, 15) dont les faces intérieures sont pourvues d'un filet (16) pour l'insertion et le blocage d'un bouchon fileté (30), **caractérisé en ce que** l'élément d'ancrage d'os comprend au moins un élément élastique qui limite localement la largeur du canal (13) et empêche ou entrave l'expulsion des tiges (5-10) du réceptacle (11) lorsqu'elles sont insérées dans la tête (4) et ne sont pas encorebloquées par le bouchon (30).

2. Élément d'ancrage d'os selon la revendication 1, comprenant au moins un siège (17) formé dans la tête (4) faisant face au canal (13) et au réceptacle (11), et un élément élastique en forme de U (18) qui peut être inséré dans ledit siège (17) et comprend deux branches verticales (19, 20) reliées par une partie inférieure sensiblement semi-circulaire (2) dont les dimensions internes et la forme correspondent sensiblement à celles du réceptacle (11), lesdites branches verticales (19, 20) comprenant chacune une protubérance (28, 29) qui réduit localement la largeur de l'espace séparant les branches (19, 20).

3. Élément d'ancrage d'os, selon la revendication2, dans lequel les bords latéraux supérieurs (22, 23) dudit siège (17) sont courbésvers l'intérieur du siège (17) de manière à réduire sa largeur au niveau de son entrée, et dans lequel les parties supérieures des branches (19, 20) du composant en forme de U (18) comprennent des encoches (24, 25).

4. Élément d'ancrage d'os, selon la revendication1, dans lequel ledit élément élastique est formé par des parties des branches (14, 15) de la tête (4).

5. Élément d'ancrage d'os, selon la revendication1, dans lequel ladite limitation locale de la largeur du canal (13) a une longueur correspondant à moins que le diamètre de la tête (4).

6. Dispositif pour corriger des déformations de la colonne vertébrale, comprenant des éléments d'ancrage d'os qui sont implantés sur les vertèbres (3) du patient, et une pluralité de tiges (5-10) de petit diamètre qui sont insérées dans la tête (4) desdits éléments, dans lequel au moins l'un desdits éléments est du type selon l'une des revendications 1 à 4.

7. Dispositif selon la revendication 6, comprenant en outre un bouchon fileté (30) pour bloquer lesdites tiges dans la tête (4) dudit au moins un desdits éléments du type selon l'une des revendications 1 à 4.
